# EUROPEAN PATENT SPECIFICATION

(11) **EP 2 419 052 B1**
(45) Date of publication and mention of the grant of the patent: **09.07.2014**
(21) Application number: 10714786.0
(22) Date of filing: 14.04.2010
(51) Int. Cl.: A61F 2/44

(54) **MINIMALLY INVASIVE EXPANDABLE VERTEBRAL IMPLANT**
MINIMAL INVASIVES EXPANDIERBARES WIRBELIMPLANTAT
IMPLANT VERTÉBRAL DILATABLE TRÈS PEU INVASIF

(30) Priority: 16.04.2009 US 424941
(43) Date of publication of application: 22.02.2012
(73) Proprietor: Warsaw Orthopedic, Inc., Warsaw, IN 46581 (US)
(72) Inventor: DVORAK, Marcel, Vancouver British Columbia V65 IN3 (CA); FISHER, Charles G., Vancouver British Columbia VGN Y2 (CA); MELKENT, Anthony J., Memphis Tennessee 38111 (US); MILLER, Keith E., Germantown Tennessee 38138 (US); RAMPERSAUD, Y. Raja, Toronto Ontario M6S 1G6 (CA); SEARS, William R., Warrawee New South Wales 2074 (AU)
(74) Representative: Viering, Jentschura & Partner Patent- und Rechtsanwälte
(86) International application number: PCT/US2010/031077
(87) International publication number: WO 2010/120915

(56) References cited:
- WO-A2-2006/060482
- US-A- 4 932 975
- US-A1- 2002 111 688
- US-A1- 2008 058 931
- US-A1- 2008 065 076

## Description

### BACKGROUND

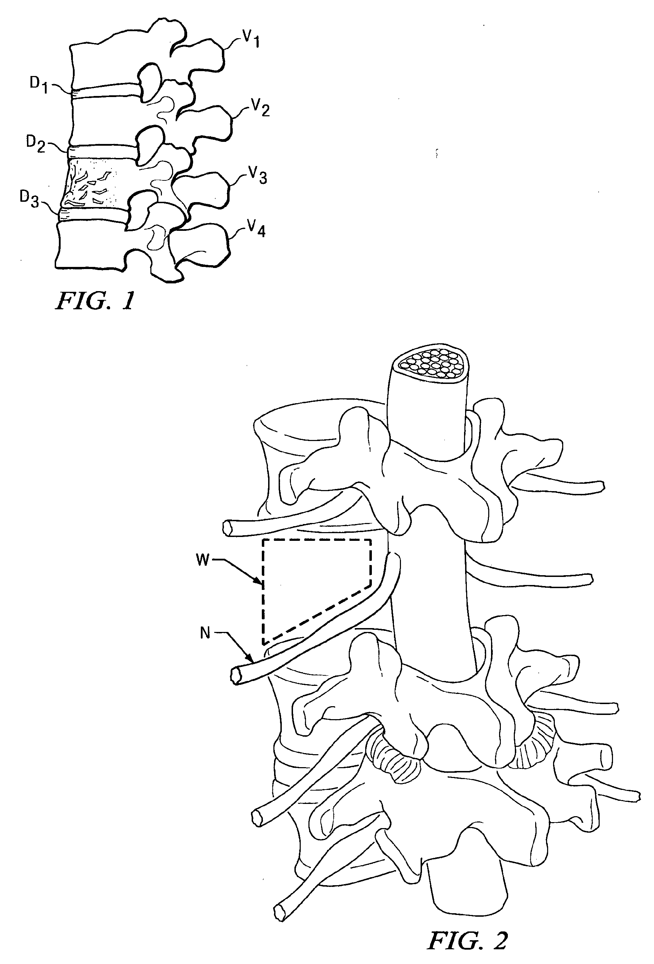

It is sometimes necessary to remove one or more vertebrae, or a portion of the vertebrae, from the human spine in response to various pathologies. For example, one or more of the vertebrae may become damaged as a result of tumor growth, or may become damaged by a traumatic or other event. Removal, or excision, of a vertebra may be referred to as a vertebrectomy. Excision of a generally anterior portion, or vertebral body, of the vertebra may be referred to as a corpectomy. An implant is usually placed between the remaining vertebrae to provide structural support for the spine as a part of a corpectomy or vertebrectomy. FIG. 1 illustrates four vertebrae, V₁-V₄ of a typical lumbar spine and three spinal discs, D₁-D₃. As illustrated, V₃ is a damaged vertebra and all or a part of V₃ could be removed to help stabilize the spine. If removed along with spinal discs D₂ and D₃, an implant may be placed between vertebrae V₂ and V₄. In some cases, the implant inserted between the vertebrae is designed to facilitate fusion between remaining vertebrae. In other cases, especially when treating tumors, the ultimate goal of the procedure is spinal stability, regardless of fusion. A successful procedure may decrease pain, preserve or enhance neurological function and allow a patient greater mobility without an external orthosis. Sometimes an implant is designed to replace the function of the excised vertebra and discs. All or part of more than one vertebra may be damaged and require removal and replacement in some circumstances. If only a portion of a vertebral body and adjacent discs are removed and replaced, the procedure is called a hemi-vertebrectomy.

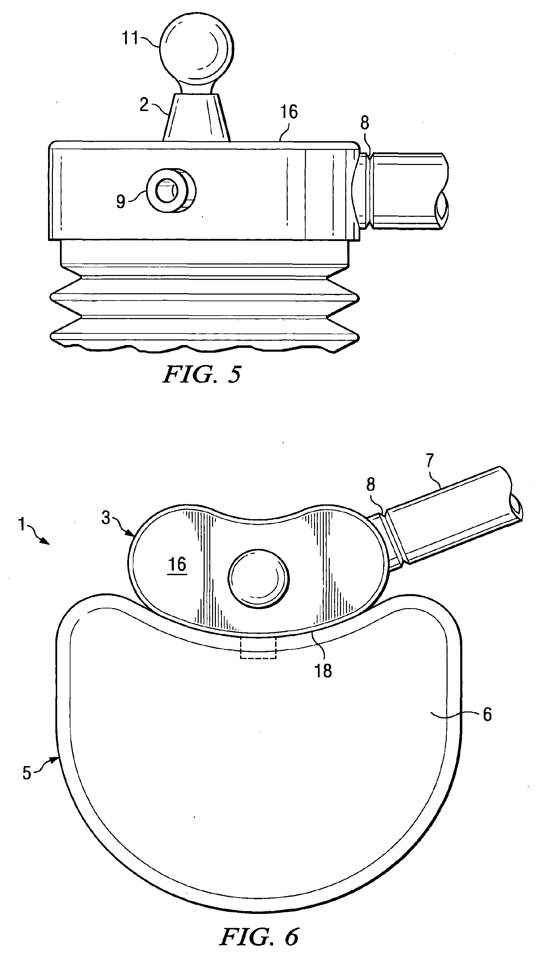

Many implants are known in the art for use in vertebrectomy and corpectomy procedures. A spinal mobility preservation apparatus is known e.g. from US 2008/0065076 A1. US 2008/0065076 A discloses in figures 5 to 7 a mobility preservation apparatus comprising a proximal body, an intermediate body, a distal body and an expandable membrane including first and second retainer rings securing the membrane to the proximal and distal body and surrounding the intermediate body. The intermediate body includes a flexible element to permit a limited degree of movement between the relative positions of the proximal body and the distal body. Furthermore, the intermediate body defines fenestrations through which a prosthetic nucleus material may be inserted into an expansion chamber defined by the expandable membrane. One class of implants is sized to directly replace the vertebra or vertebrae that are being replaced, without in situ expansion. Another class of implants is inserted in a collapsed state and then expanded once properly positioned. Expandable implants may be advantageous because they allow for a smaller incision and entry path when positioning an implant. A smaller incision may be particularly useful with a posterior approach, as illustrated in Fig 2. FIG. 2 is an illustration from the posterior of a portion of a human spine, with one thoracic vertebra removed. To support the remaining vertebral structure, an implant may be placed through the window W, avoiding the nerve root N. The nerve root N, may be mobilized to increase the size of the window W slightly, but excess movement may risk damage to the nerve root N. Therefore, for a posterior approach, an initially small expandable implant may have particular utility. A posterior approach may be preferred for patients with circumferential tumors or for patients more susceptible to the risks associated with a more extensive anterior approach. Similarly, initially small implants enabling minimal tissue disruption may be useful from any surgical approach to reduce trauma to surrounding tissues and to enhance patient recovery.

Once in position and expanded, it may be advantageous for a corpectomy or vertebrectomy implant to, as nearly as possible, fill the space vertically between the remaining vertebrae and laterally among the remaining soft tissues. Lateral expansion may increase the contact area between the implant and vertebral endplates. This expansion may reduce the potential for subsidence of the device into the adjacent vertebrae. However, it may be important that the lateral expansion not impinge on the spinal cord or nerve roots. In some instances, it may be useful to control lateral expansion to a particular distance or volume.

Expandable implants may also be useful in replacing long bones or portions of appendages such as the legs and arms, or a rib or other bone that is generally, though not necessarily, longer than it is wide. Examples include, but are not limited to a femur, tibia, fibula, humerus, radius, ulna, phalanges, clavicle, and any of the ribs. Use of the mechanisms described and claimed herein are equally applicable to treatment or repair of such bones or appendages. Similarly, expandable implants may be useful in at least some spinal fusion procedures where a spinal disc is replaced without replacing a vertebral body.

### SUMMARY

The invention provides an expandable medical implant according to claim 1. Further embodiments are described in the dependent claims.

### BRIEF DESCRIPTION OF THE DRAWINGS

FIG. 1 is an elevation view of a segment of a lumbar spine.
FIG. 2 is a posterior perspective view of a portion of a human spine.
FIG. 3 is a perspective view of an embodiment of an expandable medical implant.
FIG. 4 is a perspective view of the laterally rigid component embodiment of FIG. 3.
FIG. 5 is an enlarged perspective view of the implant of FIG. 4.
FIG. 6 is a plan view of the superior end of the implant of FIG. 3.
FIG. 7 is a plan view of the inferior end of the implant of FIG. 3.
FIG. 8 is a plan view of the superior end of the implant of FIG. 4.
FIG. 9 is a plan view of the superior end of the implant of FIG. 4 with a portion of the end material removed to illustrate the interior or the implant.
FIG. 10 is a perspective view of an embodiment of an expandable medical implant.
FIG. 11 is a plan view of the inferior end of the implant of FIG. 10.
FIG. 12 is a perspective view of an embodiment of an expandable medical implant.
FIG. 13 is a plan view of the superior end of an embodiment of an expandable medical implant.
FIG. 14 is a perspective view of an embodiment of an expandable medical implant.
FIG. 15 is a plan view of the superior end of the implant of FIG. 14.
FIG. 16 is a perspective view of the expandable medical implant of FIG. 3 in a state prior to expansion.
FIG. 17 is a perspective view of the laterally rigid component embodiment of FIG. 16.
FIG. 18 is a perspective view illustrating an embodiment of an expandable medical implant being introduced through a generally posterior approach.
FIG. 19 is a perspective view illustrating an embodiment of an expandable medical implant expanded between vertebrae.

### DETAILED DESCRIPTION

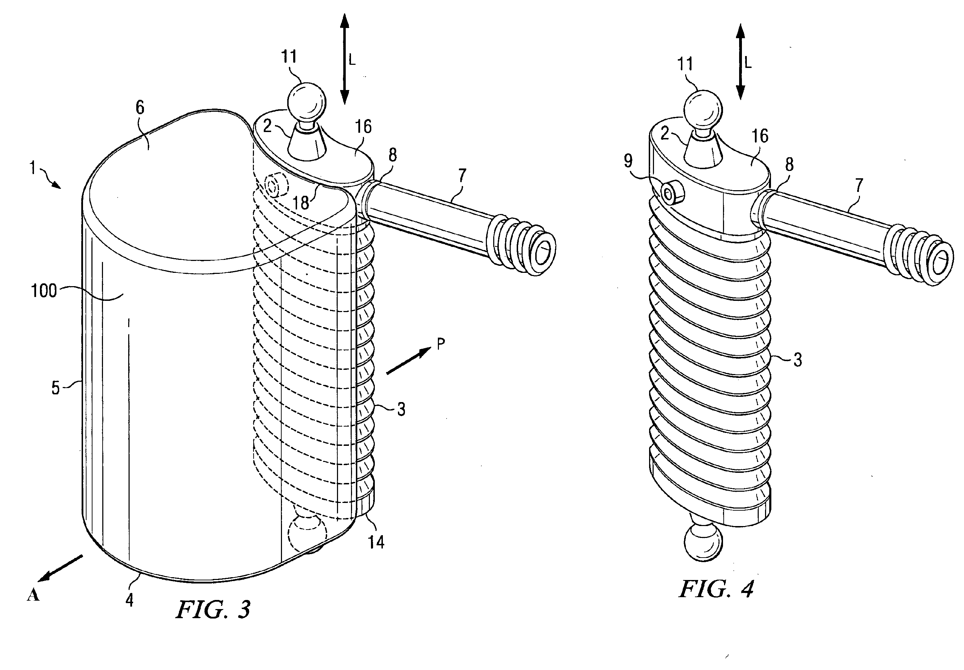

FIGS. 3-9 illustrate one embodiment of an expandable medical implant 1 for supporting skeletal structures. The expandable medical implant 1 shown includes a laterally rigid component 3 and a membrane 5. The illustrated laterally rigid component 3 is a bellows. In some embodiments, the expandable medical implant 1 will be oriented with the membrane 5 toward the anterior of the spinal column, as noted by the letter A, and with the laterally rigid component 3 toward the posterior of the spinal column, as noted by the letter P. The specified orientations are for illustrative purposes only and may be altered in various other embodiments. The longitudinal axis, or linear expansion direction L, of the laterally rigid component 3 is also illustrated. In FIGS. 3 and 4, the laterally rigid component 3 is shown in an expanded state. Expansion of the laterally rigid component 3 may be realized by introduction of a fluid into the laterally rigid component 3, may result from elasticity in the material from which the component is made, from internal or external biasing devices, or from any other effective device for generating expansion.

As shown in FIGS. 3, 4, and 6-9, the cross-sectional shape of the embodiment of the laterally rigid component 3 is concave-convex. However, in other embodiments, the cross-sectional shape of the laterally rigid component 3 may be any functional shape, such as but not limited to, generally round, oval, rectangular, triangular, polygonal, or combinations of these shapes.

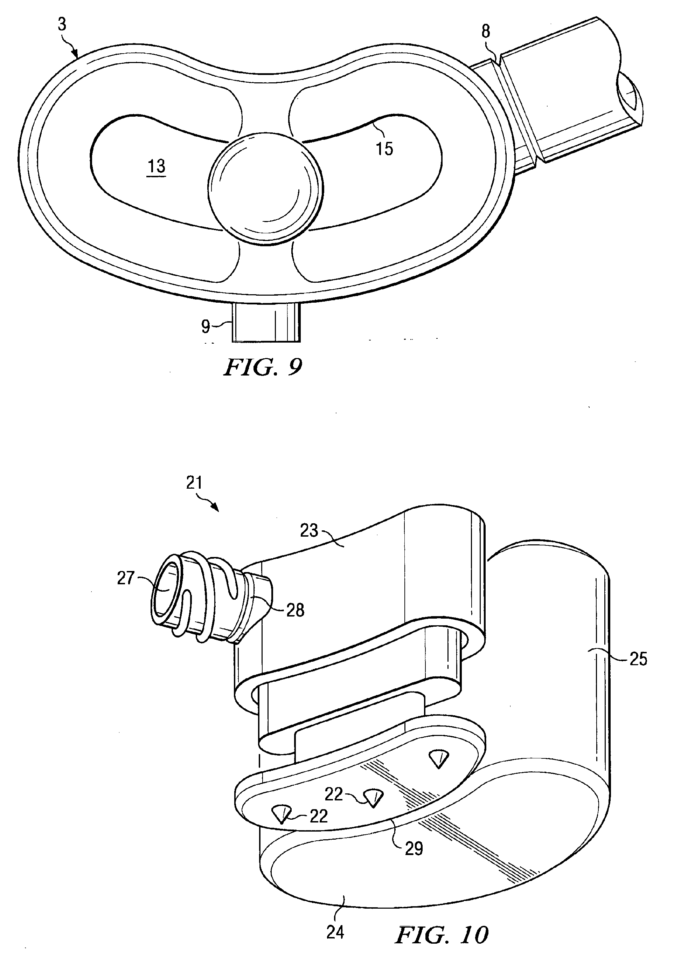

The laterally rigid component 3 illustrated in FIG. 4 is defined herein as "laterally rigid" because it appreciably resists forces applied lateral to its linear axis L. Other devices that expand linearly and resist forces applied laterally to their linear axes L, are telescoping devices of FIGS. 10-15. These, and any other devices that provide resistance to lateral forces, are contemplated in embodiments of the present invention and are within the scope of the claims herein. Other devices that may meet these limitations include, but are not limited to, reinforced elastomeric bodies, such as a polymer body impregnated with or in combination with reinforcing fibers, or passively expanding bodies driven by the expansion of other connected components. Resistance to lateral forces may be useful in the illustrated embodiments for protecting posterior neural structures such as, but not limited to, the spinal cord, spinal canal, and nerve roots.

FIGS. 3-5 illustrate a nozzle 2 extending from the laterally rigid component 3. A similar nozzle may, alternatively or in addition, extend from the opposite end of the laterally rigid component 3. The illustrated nozzle 2 is open to the interior of the laterally rigid component 3. A balloon 11 is shown extending from an open, distal end of the nozzle 2. The balloon 11 shown is in fluid communication with the interior of the laterally rigid component 3. The nozzle 2 and balloon 11 of some embodiments are configured to extend from the laterally rigid component 3 and into an endplate of an adjacent vertebra. The balloon 11 may be filled with a material, such as a flowable material, to assist in attachment of the laterally rigid component 3 to the adjacent vertebra. In some embodiments, the balloon 11 is filled separately from the laterally rigid component 3 or the membrane 5. The balloon 11 may additional have a therapeutic effect on the vertebra. For example, and without limitation, the balloon 11, alone or in combination with the nozzle 2, may help to stabilize the vertebra. The material used to inflate the balloon 11 may be a curable material or may be a material that is used to expand the balloon 11, but does not cure in place. Once expanded, the balloon 11 may also receive additional materials that permanently fill the balloon 11, or that have an additional therapeutic effect on the vertebra. Any of the materials for use in the balloon 11 may also be a fill material 100 as described in detail below. The balloon 11 may in whole or in part be non-permeable, semi-permeable, or permeable to allow the flowable material or the fill material 100 to contact the vertebra. In addition to or instead of the nozzle 2 or balloon 11, an end of an embodiment of the laterally rigid component 3 my include teeth, spikes, ridges, indentations, roughening, knurling, or any other device for enhancing fixation between a vertebra and the laterally rigid component 3. The embodiments of FIGS. 10-15 illustrate spikes 22 on the end of laterally rigid components.

The membrane 5 is illustrated in an expanded configuration in FIG. 3. The membrane 5 defines a volume and has an upper surface 6 and an opposite lower surface 4. The membrane 5 is shown coupled laterally adjacent to the laterally rigid component 3. The illustrated membrane 5 and the laterally rigid component 3 are adjacent, with the volume defined by the membrane 5 exclusive of the laterally rigid component 3, as defined herein. In some embodiments, it is advantageous for the membrane 5 and the laterally rigid component 3 to be configured in different volumes such that expansion of the respective devices may be controlled independently or enacted sequentially or in parallel. The membrane 5 of some embodiments is configured to be placed between vertebrae and expanded such that the upper surface 6 contacts a first vertebra and the opposite lower surface 4 contacts a second vertebra to provide support between the vertebrae. Lateral expansion of the membrane 5 is also accomplished in some embodiments. For example, in FIG. 3 anterior expansion, as well as medial-lateral expansion, and intervening radial expansions, are illustrated. As used herein, the term lateral means directions approximately normal to the linear expansion direction L.

The membrane 5 may be constructed, in whole or in part, of a non-permeable material. The membrane 5 may include compliant or non-compliant balloon materials such as those commonly used to manufacture coronary and Kyphoplasty medical devices. Such materials may include, but not be limited to, mylar, rubber, polyurethane, vinyl, latex, polyethylenes, ionomer, and polytetrapthalate (PET), as well as less flexible materials such as Kevlar®, PEBAX®, stainless steel, titanium, nickel-titanium alloys, and other metals and alloys and/or ceramics. A compliant membrane may include reinforcing to limit one or both of the size and shape of the membrane to a clinically advantageous extent. A non-compliant membrane may expand more elastically to more completely fill an irregular opening, depending on the amount of material introduced into the membrane.

Likewise the membrane 5 may be constructed, in whole or in part, of a permeable material, which allows a certain amount of fill material 100 to pass through the membrane 5. All or a portion may be made permeable by fabricating a material, including but not limited to, the membrane materials listed above, into a fabric, weave, mesh, composite, bonded fiber assembly, or any other manufacture known to those skilled in the art. For example, all or part of the upper surface 6 and the opposite lower surface 4 may be constructed of a permeable material to allow fill material 100 to move through the membrane 5 and to come into contact with vertebrae.

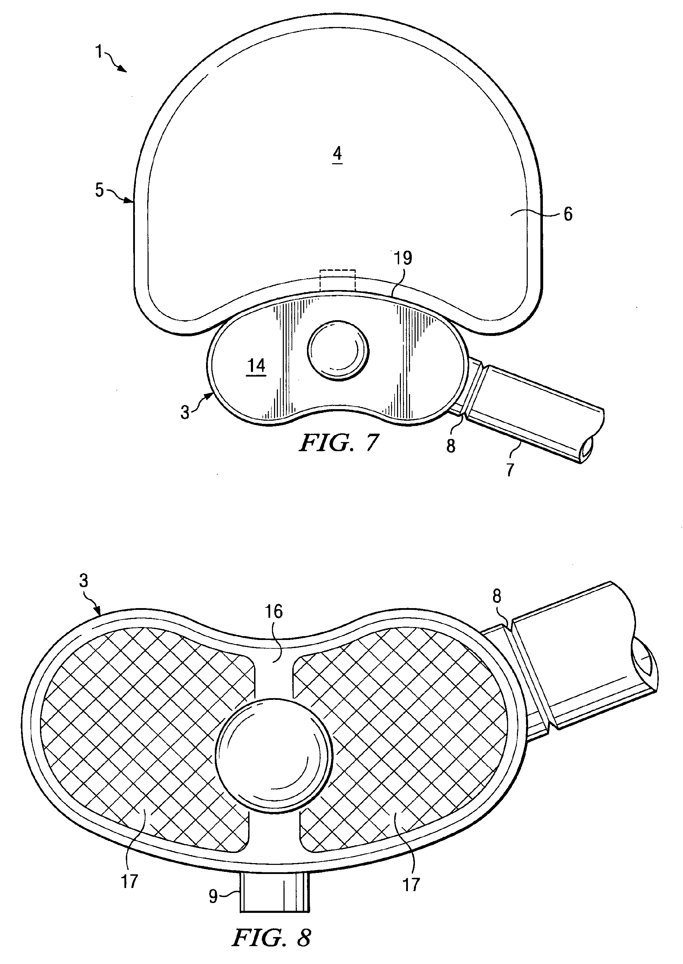

FIGS. 6 and 7 illustrate, respectively, superior and inferior ends of an expanded expandable medical implant 1. The laterally rigid component 3 shown includes an upper cap 16 and a lower cap 14. The upper and lower caps 16, 14 may be non-permeable or permeable to some degree, with similar functions to, and made from similar materials with, the membrane 5 discussed above. The upper and lower caps 16, 14 may be made from the same material as the rest of the laterally rigid component 3, or may be made from a separate material. FIG. 8 illustrates an upper cap 16 made from an at least partially permeable woven or mesh material. As illustrated, two mesh ends 17 are integrated into the upper cap 16 to allow passage of some of the fluid or fill material 100 to contact an adjacent vertebra. Alternately, or in addition, the upper cap 16 may be expanded or stretched into contact with an adjacent vertebra to conform to the contours of the vertebral endplate so as to provide an improved interface between the expandable medical implant 1 and the vertebra. FIG. 9 shows the upper end cap 16 of FIG. 8 with the two mesh ends 17 removed to illustrate the interior of an embodiment of the laterally rigid component 3. The interior shown includes a chamber 13 which is open along the length of the laterally rigid component 3 between the upper and lower caps 16, 14. In this embodiment, the chamber 13 is open whether the laterally rigid component 3 is in an expanded or unexpanded state. The interior edge 15 of the bellows defines an inner periphery of the chamber 13. In some embodiments, elasticity in the material along the interior edges 15 of the bellows may sever as a biasing force to bias the laterally rigid component 3 toward an expanded or unexpanded state, as may be advantageous in various circumstances. For example, it may be advantageous to bias the laterally rigid component 3 toward an unexpanded state to provide a low profile device for insertion. Other devices may be used to expand the device as needed. In other circumstances, it may be preferred to bias the laterally rigid component 3 toward an expanded state. With such an embodiment, another component may be used to keep the laterally rigid component 3 in an unexpanded state while it is inserted. Then following insertion, the laterally rigid component 3 may be released and allowed to increase toward its expanded state.

FIGS. 6 and 7 illustrate the membrane 5 being coupled laterally adjacent to the laterally rigid component 3. In the embodiment of FIG. 6, an adhesive may be applied between the membrane 5 and the laterally rigid component 3 at a first point 18 near the upper surface 6 of the membrane 5. As illustrated in FIG. 7, the membrane 5 may be coupled to the laterally rigid component 3 with an adhesive applied at a second point 19 near the lower surface 4 of the membrane 5.

Embodiments of the expandable medical implant 1 also include a port 7 for receiving a fluid. As used in association with this function, a fluid may be a paste, gel, liquid, suspension, granular mixture, or similar substance. A substance as described herein will be considered a fluid even if it later cures or hardens to a non-fluidic state. As illustrated in FIGS. 3-9, the port 7 is connected to the laterally rigid component 3. In other embodiments, the port 7 may connect to the membrane 5, or to another portion of the expandable medical implant 1. The fluid received through the port 7 in some circumstances will drive expansion of the expandable medical implant 1. This may be accomplished by pressurizing the laterally rigid component 3 or the membrane 5, or by pressurizing both. In other embodiments, the fluid may be used to maintain expansion generated by some other force such as expansion from elasticity in the material from which the laterally rigid component 3 or membrane 5 are made, from biasing devices internal or external to the laterally rigid component 3 or membrane 5, or from any other effective device for generating expansion. The illustrated port 7 includes a relief 8 that may be useful in detaching the port 7 from the laterally rigid component 3 after expansion of the implant, but prior to completion of the surgical procedure. The relief 8 provides both a tactile indication of an appropriate location to detach the port 7 and a reduced material thickness that allows for less resistance to cutting, breaking, or otherwise removing the port 7. The port 7 may also serve as an extension of the expandable medical implant 1 useful in manipulating the expandable medical implant 1 into a clinically effective location.

As shown in FIGS. 4, 5, 8, and 9, a transfer opening 9 is provided between the laterally rigid component 3 and the membrane 5. In some embodiments, the transfer opening 9 is a hole through which a fluid or fill material 100, or both, may pass. Passage may occur in either direction between the laterally rigid component 3 and the membrane 5 in various embodiments. As illustrated, fluid, fill material 100 or both enter the laterally rigid component 3 through the port 7 and pass through the transfer opening 9 into the membrane 5. In some embodiments, a valve is provided at the transfer opening 9 to control flow between the laterally rigid component 3 and the membrane 5. The valve may be controlled by direct manipulation or through instrumentation connected through the port 7. The valve may also be pressure actuated such that flow is allowed through the transfer opening 9 above a certain pressure.

The fill material 100 may enter the expandable medical implant 1 as a fluid, and then harden or cure in the implant. In some embodiments, a non-hardenable and non-curing fluid is used to expand, or to hold expansion in, the implant or one or some of the components of the implant. A fill material 100 may then be introduced into at least the membrane 5 to provide support between the upper surface 6 and the lower surface 4. The fill material 100 may be a paste, gel, liquid, suspension, granular mixture, or similar substance. Non-limiting examples of fill materials 100 include bone cement, paste, morselized allograft, autograft, or xenograft bone, ceramics, or various polymers. An example bone cement is polymethylmethacrylate (PMMA), which may be made from methylmethacrylate, polymethylmethacrylate, esters of methacrylic acid, or copolymers containing polymethylmethacrylate and polystyrene. Additional non-limiting examples of the fill material 100 include semi-rigid flowable or hardenable material such as silicone or various types of urethane materials. It should further be understood that other types of fill materials 100 which are not necessarily hardenable or curable may be used in association with the present invention. For example, the fill material may comprise beads or small particles or grains of material, some of which may, in aggregate, achieve a harder consistency as a result of interlocking or compaction. In some embodiments, the fill material may also include a bone growth promoting substance. The use and components of such bone growth promoting substances are described in more detail below.

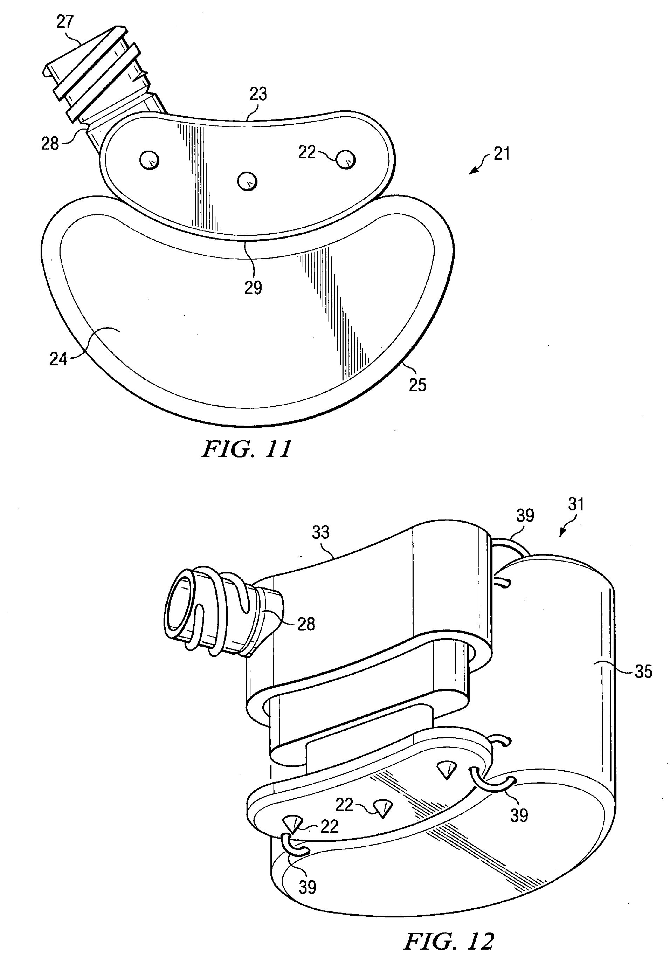

FIGS. 10 and 11 show another embodiment of an expandable medical implant 21. An inferior side of the implant is illustrated in FIG. 11, as well as in FIG. 10. The expandable medical implant 21 shown includes a laterally rigid component 23 and a membrane 25. The illustrated laterally rigid component 23 is a telescoping body with spikes 22 for enhanced connection to an adjacent vertebral body. In some embodiments, the expandable medical implant 21 will be oriented with the membrane 25 toward the anterior of the spinal column and with the laterally rigid component 23 toward the posterior of the spinal column. The specified orientations are for illustrative purposes only and may be altered in various other embodiments. The laterally rigid component 23 is shown in an expanded state. Expansion of the laterally rigid component 23 may be realized by introduction of a fluid into the laterally rigid component 23, may result from a biasing device resident in the laterally rigid component 23, or from any other effective device for generating expansion. The membrane 25 is essentially similar to the membrane 5 described in greater detail above. A port 27 coupled to the laterally rigid component 23 is also illustrated. A relief 28 is depicted that provides both a tactile indication of an appropriate location to detach the port 27 and a reduced material thickness that allows for less resistance to cutting, breaking, or otherwise removing the port 27. Similar to the previous embodiment, an adhesive may be applied between the membrane 25 and the laterally rigid component 23. FIGS. 10 and 11 illustrate an embodiment where the adhesive may be applied at a point 29 near a lower surface 24 of the membrane 25.

FIG. 12 illustrates an embodiment of an expandable medical implant 31 with a membrane 35 coupled to a laterally rigid component 33 by a number of tethers 39. Each tether 39 may be made of any biocompatible material. The tethers 39 illustrated are strands, but in other embodiments may be loops, hooks, staples, fasteners of any kind, or any other component capable of connecting the membrane 35 with the laterally rigid component 33. The operation and structures of the embodiment of FIG. 12 are otherwise essentially similar to the operation and structures of the other similar embodiments described herein.

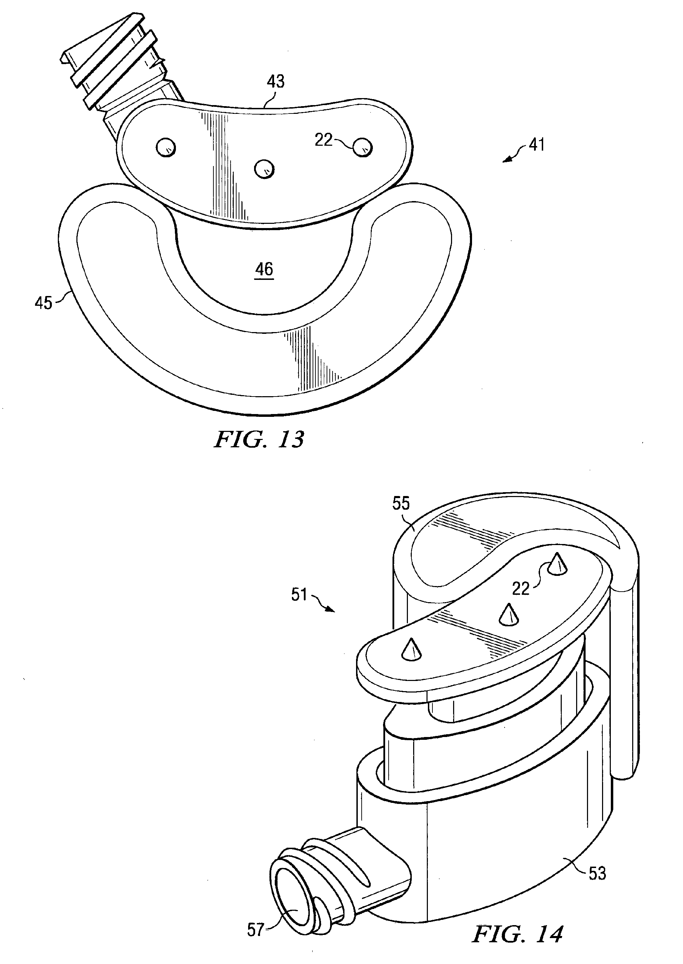

FIG. 13 illustrates an embodiment of an expandable medical implant 41 with a membrane 45 coupled to a laterally rigid component 43 such that a void 46 is created between the membrane 45 and the laterally rigid component 43. The membrane 45 may be coupled to the laterally rigid component 43 by any effective mechanism, including but not limited to an adhesive or tether. The void 46 may be filled with an osteogenic material or therapeutic composition. Filling may occur through an opening into the void 46 through the membrane 45, the laterally rigid component 43, or otherwise. Filling may be through a syringe or similar device, through direct placement, or by any other effective mechanism. Osteogenic materials include, without limitation, autograft, allograft, xenograft, demineralized bone, synthetic and natural bone graft substitutes, such as bioceramics and polymers, and osteoinductive factors. A separate carrier to hold materials within the device may also be used. These carriers may include collagen-based carriers, bioceramic materials, such as BIOGLASS®, hydroxyapatite and calcium phosphate compositions. The carrier material may be provided in the form of a sponge, a block, folded sheet, putty, paste, graft material or other suitable form. The osteogenic compositions may include an effective amount of a bone morphogenetic protein (BMP), transforming growth factor β1, insulin-like growth factor, platelet-derived growth factor, fibroblast growth factor, LIM mineralization protein (LMP), and combinations thereof or other therapeutic or infection resistant agents, separately or held within a suitable carrier material. The operation and structures of the embodiment of FIG. 13 are otherwise essentially similar to the operation and structures of the other similar embodiments described herein.

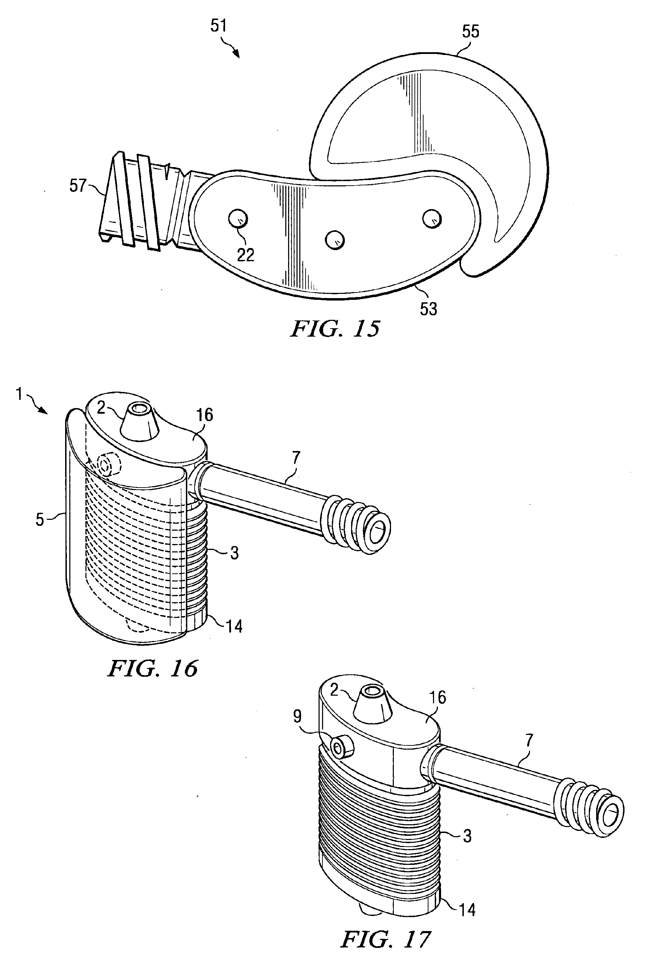

FIGS. 14 and 15 illustrate an embodiment of an expandable medical implant 51 with a membrane 55 coupled to a laterally rigid component 53. The operation and structures of the embodiment of FIGS. 14 and 15 are essentially similar to the operation and structures of the other similar embodiments described herein, except that the membrane 55 is sized or otherwise constrained such that it will only fill one lateral side of a vertebral space into which it is placed. The contralateral side of such a vertebral space may be left open for an additional implant, or may include healthy bone material and not need to be excised or filled. The membrane 55 may include layers, internal fibers or structures, or non-expandable materials that limit its expansion to one or both of a specific size and shape. The embodiment of FIGS. 14 and 15 shows a port 57 near an inferior end of the laterally rigid component 53 such that the implant may be expanded from an inferior vertebra toward a superior vertebra. Note that any of the embodiments of the invention disclosed herein may be deployed from an inferior, superior, or even medial position effectively and within the claims herein. In some embodiments, the size or shape of the membrane 55 may be limited to only fill any particular portion of a vertebral space, not necessarily just a lateral portion.

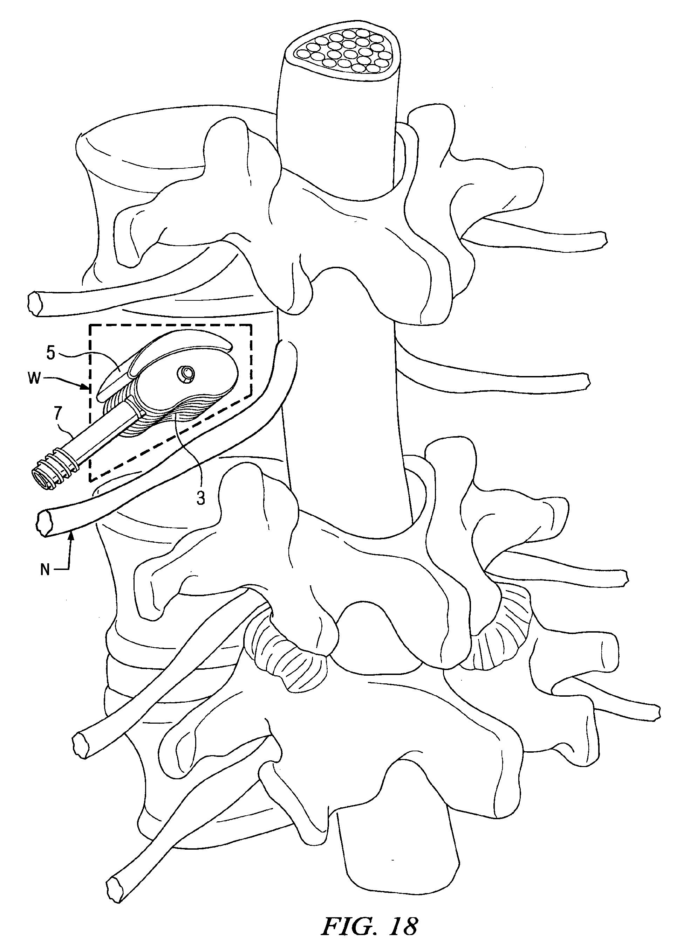

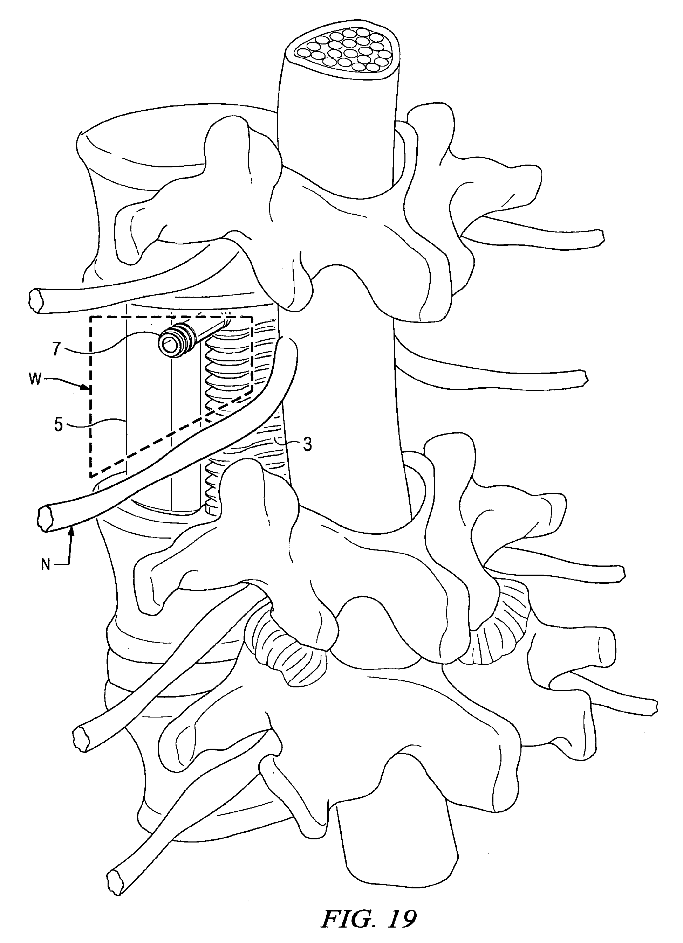

FIGS. 16 and 17 show the expandable medical implant 1 of FIG. 3 in an unexpanded or contracted state. Both the laterally rigid component 3 and the membrane 5 are unexpanded linearly. The membrane 5 is unexpanded laterally as well as linearly. The port 7 may be used to handle the expandable medical implant 1 or to guide the implant into a position where it can be effectively deployed. One manipulation of an embodiment of the expandable medical implant 1 is illustrated in FIGS. 18 and 19.

Each of the embodiments disclosed herein may be described as a means for occupying a vertebral space. This may be accomplished with a linearly expandable means for occupying at least a portion of a space between vertebrae and for providing protection to neural structures in combination with a laterally expandable means coupled adjacent to the linearly expandable means. The laterally expandable means of some embodiments is for receiving and containing a fill material between the linearly expandable means posteriorly and soft tissues surrounding a spinal column. The soft tissues surrounding the spinal column may include, but are not limited to, one or more of ligaments, muscles, vessels, arteries, and neural structures.

Embodiments of the implant in whole or in part may be constructed of biocompatible materials of various types. Examples of implant materials include, but are not limited to, non-reinforced polymers, carbon-reinforced polymer composites, PEEK and PEEK composites, low density polyethylene, shape-memory alloys, titanium, titanium alloys, cobalt chrome alloys, stainless steel, ceramics and combinations thereof. If a trial instrument or implant is made from radiolucent material, radiographic markers can be located on the trial instrument or implant to provide the ability to monitor and determine radiographically or fluoroscopically the location of the body in the spinal space. In some embodiments, the implant or individual components of the implant may be constructed of solid sections of bone or other tissues. Tissue materials include, but are not limited to, synthetic or natural autograft, allograft or xenograft, and may be resorbable or non-resorbable in nature. Examples of other tissue materials include, but are not limited to, hard tissues, connective tissues, demineralized bone matrix and combinations thereof.

FIG. 1 illustrates four vertebrae, V₁-V₄, of a typical lumbar spine and three spinal discs, D₁-D₃, and FIG. 2 depicts a portion of a typical thoracic spine. Embodiments of the invention may be applied to the lumbar spinal region, and embodiments may also be applied to the cervical or thoracic spine or between other skeletal structures.

Some embodiments may also include supplemental fixation devices in addition to or as part of the expandable medical implant for further stabilizing the anatomy. For example, and without limitation, rod and screw fixation systems, anterior, posterior, or lateral plating systems, facet stabilization systems, spinal process stabilization systems, and any devices that supplement stabilization may be used as a part of or in combination with the expandable medical implant. Embodiments of the invention may be useful in at least some spinal fusion procedures where a spinal disc is replaced without replacing a vertebral body.

FIGS. 18 and 19 illustrate the introduction and expansion of an expandable medical implant into a vertebral space. The implant is placed through the window W, as described above, and past a nerve root N. The window W is created among the soft tissues of a segment of the spinal column. The expandable medical implant introduced is a vertebral prosthesis that includes the laterally rigid expandable component 3 and the adjacent membrane 5 with an upper surface and an opposite lower surface. FIG. 18 shows a distal end of the expandable medical implant being introduced through the window W first. In other embodiments, another portion of the expandable medical implant may be introduced first and other translational and rotational manipulations may be conducted on the implant as it is introduced.

Once the expandable medical implant has been introduced through the window W and positioned appropriately in the vertebral space, it may be expanded linearly and laterally. A linearly and laterally expanded implant is illustrated in FIG. 19. The embodiment illustrated may be expanded by introducing a fluid through the port 7 and into the laterally rigid component 3. Introducing fluid through the port 7 may be caused by pressure or force generated by a syringe, injector, multi-stage injector, central pressurization reservoir, or any effective system or device. The fluid introduced to expand the laterally rigid component 3 may be fluid that is introduced only to expand the component, or in some instances, is a final fill material 100. When the fluid introduce to expand the implant is not, or is not a component of, the fill material 100, the fluid may be removed from the implant after the fill material 100 has been introduced. The introduced fluid may be one part of a multi-part fill material. The fluid or a fill material 100 may further be passed from the laterally rigid component 3 into the membrane 5 to linearly and laterally expand the membrane 5. In some embodiments, flow into the laterally rigid component 3 and the membrane 5 are separately controlled so that one or the other may be selectively filled independent from the other. In some embodiments, an additional port, or a port replacing the port 7 in the laterally rigid component 3, may connect with the membrane 5 directly.

In some embodiments, introduction of the fill material 100, by whatever mechanism that is effective, results in a non-fluidic structural support between the upper and lower surfaces of the membrane 5. Even though a fill material 100 may be initially introduced as a fluid, embodiments of the invention provide for the fill material 100 to cure or harden as noted above and provide structural support. Other fill materials 100 may have a non-fluidic state as a result of interlocking of the particles with one another or with the membrane 5, or may be non-fluidic as a response to compaction.

The expandable medical implant is shown in FIGS. 18 and 19 as being implanted from a generally posterior approach. However, embodiments of the invention may include implantation from any surgical approach, including but not limited to, posterior, lateral, anterior, transpedicular, lateral extracavitary, in conjunction with a laminectomy, in conjunction with a costotransversectomy, or by any combination of these and other approaches.

Terms such as lower, upper, anterior, posterior, inferior, superior, lateral, medial, contralateral, and the like have been used herein to note relative positions. However, such terms are not limited to specific coordinate orientations, but are used to describe relative positions referencing particular embodiments. Such terms are not generally limiting to the scope of the claims made herein.

While embodiments of the invention have been illustrated and described in detail in the disclosure, the disclosure is to be considered as illustrative and not restrictive in character. All changes and modifications that come within the scope of the invention are to be considered within the scope of the disclosure.

## Claims

1. An expandable medical implant (1, 21, 31, 41, 51) for supporting skeletal structures comprising:
a laterally rigid component (3, 23, 33, 43, 53) that is longitudinally expandable;
a membrane (5, 25, 35, 45, 55) defining a volume and having an upper surface (6) and an opposite lower surface (4), the membrane (5, 25, 35, 45, 55) coupled adjacent to the laterally rigid component (3, 23, 33, 43, 53) ; and
a port (7, 27, 57) in the expandable medical implant (1, 21, 31, 41, 51) for receiving a fluid that drives or maintains expansion of the expandable medical implant (1, 21, 31, 41, 51) ;
wherein the volume defined by the membrane (5, 25, 35, 45, 55) is exclusive of the laterally rigid component (3, 23, 33, 43, 53).

2. The expandable medical implant (1) of claim 1 wherein the laterally rigid component (3)is a linearly expandable bellows.

3. The expandable medical implant (21) of claim 1 wherein the laterally rigid component (23) is a telescoping body.

4. The expandable medical implant (1, 21, 31, 41, 51) of claim 1 wherein the membrane is, at least in part, a non-permeable membrane (5, 25, 35, 45, 55).

5. The expandable medical implant (1, 21, 31, 41, 51) of claim 1 wherein the membrane is, at least in part, a permeable membrane (5, 25, 35, 45, 55).

6. The expandable medical implant (1, 21, 31, 41, 51) of claim wherein the membrane (5, 25, 35, 45, 55) is laterally expandable.

7. The expandable medical implant (1, 21, 31, 41, 51) of claim 1 wherein the membrane (5, 25, 35, 45, 55) is coupled laterally adjacent to the laterally rigid component (3, 23, 33, 43, 53).

8. The expandable medical implant (31) of claim 7, further comprising at least one tether (39) between the membrane (35) and the laterally rigid component (33).

9. The expandable medical implant (1, 21, 31, 41, 51) of claim 7, further comprising an adhesive between the membrane (5, 25, 35, 45, 55) and the laterally rigid component (3, 23, 33, 43, 53).

10. The expandable medical implant (1, 21, 31, 41, 51) of claim 7 wherein the membrane (5, 25, 35, 45, 55) is coupled to the laterally rigid component (3, 23, 33, 43, 53) at a first point near its upper surface (6) and at a second point near its lower surface (4).

11. The expandable medical implant (41) of claim 1 wherein the membrane (45) is coupled to the laterally rigid component (43) to create a void (46) fillable with osteogenic material between the membrane (45) and the laterally rigid component (43).

12. The expandable medical implant (51) of claim 1 wherein the membrane (55) is sized or constrained such that it may only fill one lateral side of a vertebral space and will not fill the contralateral side.

13. The expandable medical implant (1, 21, 31, 41, 51) of claim 1 wherein the port (7, 27, 57) in the expandable medical implant (1, 21, 31, 41, 51) is an opening in the laterally rigid component (3, 23, 33, 43, 53).

14. The expandable medical implant (1, 21, 31, 41, 51) of claim 13, further comprising a transfer opening (9) from the laterally rigid component (3, 23, 33, 43, 53) to the membrane (5, 25, 35, 45, 55).

15. The expandable medical implant (1, 21, 31, 41, 51) of claim 1, further comprising a fill material occupying at least the membrane (5, 25, 35, 45, 55) and providing support between the upper (6) and the lower (4) surface.

## Patentansprüche

1. Expandierbares medizinisches Implantat (1, 21, 31, 41, 51) zum Abstützen von Skelett-Strukturen, aufweisend
eine in Querrichtung steife Komponente (3, 23, 33, 43, 53), die in Längsrichtung expandierbar ist,
eine Membran (5, 25, 35, 45, 55), die ein Volumen definiert und eine obere Fläche (6) und eine gegenüberliegende untere Fläche (4) aufweist, wobei die Membran (5, 25, 35, 45, 55) benachbart zu der in Querrichtung steifen Komponente (3, 23, 33, 43, 53) gekuppelt ist, und
einen Anschluss (7, 27, 57) in dem expandierbaren medizinischen Implantat (1, 21, 31, 41, 51) zum Aufnehmen eines Fluides, das die Expansion des expandierbaren Implantats (1, 21, 31, 41, 51) antreibt oder diese aufrechterhält,
wobei das Volumen, das mittels der Membran (5, 25, 35, 45, 55) definiert ist, nicht die in Querrichtung steife Komponente (3, 23, 33, 43, 53) enthält.

2. Expandierbares medizinisches Implantat (1) gemäß Anspruch 1, wobei die in Querrichtung steife Komponente (3) ein linear expandierbarer Balg ist.

3. Expandierbares medizinisches Implantat (21) gemäß Anspruch 1, wobei die in Querrichtung steife Komponente (23) ein Teleskop-Körper ist.

4. Expandierbares medizinisches Implantat (1, 21, 31, 41, 51) gemäß Anspruch 1, wobei die Membran, zumindest abschnittsweise, eine nicht-permeable Membran (5, 25, 35, 45, 55) ist.

5. Expandierbares medizinisches Implantat (1, 21, 31, 41, 51) gemäß Anspruch 1, wobei die Membran, zumindest abschnittsweise, eine permeable Membran (5, 25, 35, 45, 55) ist.

6. Expandierbares medizinisches Implantat (1, 21, 31, 41, 51) gemäß Anspruch 1, wobei die Membran (5, 25, 35, 45, 55) in Querrichtung expandierbar ist.

7. Expandierbares medizinisches Implantat (1, 21, 31, 41, 51) gemäß Anspruch 1, wobei die Membran (5, 25, 35, 45, 55) in Querrichtung benachbart zu der in Querrichtung steifen Komponente (3, 23, 33, 43, 53) mit dieser gekuppelt ist.

8. Expandierbares medizinisches Implantat (31) gemäß Anspruch 7, ferner aufweisend zumindest ein Halteelement (39) zwischen der Membran (35) und der in Querrichtung steifen Komponente (33).

9. Expandierbares medizinisches Implantat (1, 21, 31, 41, 51) gemäß Anspruch 7, ferner aufweisend ein Haftmittel zwischen der Membran (5, 25, 35, 45, 55) und der in Querrichtung steifen Komponente (3, 23, 33, 43, 53).

10. Expandierbares medizinisches Implantat (1, 21, 31, 41, 51) gemäß Anspruch 7, wobei die Membran (5, 25, 35, 45, 55) an einem ersten Punkt nahe bei der oberen Fläche (6) davon und an einem zweiten Punkt nahe der unteren Fläche (4) davon mit der in Querrichtung steifen Komponente (3, 23, 33, 43, 53) gekuppelt ist.

11. Expandierbares medizinisches Implantat (41) gemäß Anspruch 1, wobei die Membran (45) mit der in Querrichtung steifen Komponente (43) gekuppelt ist, um einen Freiraum (46) zwischen der Membran (45) und der in Querrichtung steifen Komponente (43) zu erzeugen, der mit einem osteogenen Material füllbar ist.

12. Expandierbares medizinisches Implantat (51) gemäß Anspruch 1, wobei die Membran (55) solch eine Größe hat oder so gehalten wird, dass sie nur eine laterale Seite eines Vertebralraums füllen kann und nicht die kontralaterale Seite füllt.

13. Expandierbares medizinisches Implantat (1, 21, 31, 41, 51) gemäß Anspruch 1, wobei der Anschluss (7, 27, 57) in dem expandierbaren medizinischen Implantat (1, 21, 31, 41, 51) eine Öffnung in der in Querrichtung steifen Komponente (3, 23, 33, 43, 53) ist.

14. Expandierbares medizinisches Implantat (1, 21, 31, 41, 51) gemäß Anspruch 13, ferner aufweisend eine Transferöffnung (9) von der in Querrichtung steifen Komponente (3, 23, 33, 43, 53) zu der Membran (5, 25, 35, 45, 55).

15. Expandierbares medizinisches Implantat (1, 21, 31, 41, 51) gemäß Anspruch 1, ferner aufweisend ein Füllmaterial, das zumindest die Membran (5, 25, 35, 45, 55) okkupiert und eine Abstützung zwischen der oberen (6) und der unteren (4) Fläche bereitstellt.

## Revendications

1. Implant médical expansible (1, 21, 31, 41, 51) pour supporter des structures squelettiques comprenant :
un composant latéralement rigide (3, 23, 33, 43, 53) qui est longitudinalement expansible ;
une membrane (5, 25, 35, 45, 55) définissant un volume et ayant une surface supérieure (6) et une surface inférieure (4) opposée, la membrane (5, 25, 35, 45, 55) étant couplée de manière adjacente au composant latéralement rigide (3, 23, 33, 43, 53) ; et
un orifice (7, 27, 57) dans l'implant médical expansible (1, 21, 31, 41, 51) pour recevoir un fluide qui entraîne ou maintient l'expansion de l'implant médical expansible (1, 21, 31, 41, 51 ) ;
dans lequel le volume défini par la membrane (5, 25, 35, 45, 55) exclut le composant latéralement rigide (3, 23, 33, 43, 53).

2. Implant médical expansible (1) selon la revendication 1, dans lequel le composant latéralement rigide (3) est un soufflet linéairement expansible.

3. Implant médical expansible (21) selon la revendication 1, dans lequel le composant latéralement rigide (23) est un corps télescopique.

4. Implant médical expansible (1, 21, 31, 41, 51) selon la revendication 1, dans lequel la membrane est, au moins en partie, une membrane non perméable (5, 25, 35, 45, 55).

5. Implant médical expansible (1, 21, 31, 41, 51) selon la revendication 1, dans lequel la membrane est, au moins en partie, une membrane perméable (5, 25, 35, 45, 55).

6. Implant médical expansible (1, 21, 31, 41, 51) selon la revendication 1, dans lequel la membrane (5, 25, 35, 45, 55) est latéralement expansible.

7. Implant médical expansible (1, 21, 31, 41, 51) selon la revendication 1, dans lequel la membrane (5, 25, 35, 45, 55) est couplée de manière latéralement adjacente au composant latéralement rigide (3, 23, 33, 43, 53).

8. Implant médical expansible (31) selon la revendication 7, comprenant en outre au moins une attache (39) entre la membrane (35) et le composant latéralement rigide (33).

9. Implant médical expansible (1, 21, 31, 41, 51) selon la revendication 7, comprenant en outre un adhésif entre la membrane (5, 25, 35, 45, 55) et le composant latéralement rigide (3, 23, 33, 43, 53).

10. Implant médical expansible (1, 21, 31, 41, 51) selon la revendication 7, dans lequel la membrane (5, 25, 35, 45, 55) est couplée au composant latéralement rigide (3, 23, 33, 43, 53) à un premier point à proximité de sa surface supérieure (6) et à un second point à proximité de sa surface inférieure (4).

11. Implant médical expansible (41) selon la revendication 1, dans lequel la membrane (45) est couplée au composant latéralement rigide (43) afin de créer un vide (46) pouvant être rempli avec un matériau ostéogénique entre la membrane (45) et le composant latéralement rigide (43).

12. Implant médical expansible (51) selon la revendication 1, dans lequel la membrane (55) est dimensionnée ou contrainte de sorte qu'elle peut uniquement remplir un côté latéral d'un espace vertébral et ne peut pas remplir le côté controlatéral.

13. Implant médical expansible (1, 21, 31, 41, 51) selon la revendication 1, dans lequel l'orifice (7, 27, 57) dans l'implant médical expansible (1, 21, 31, 41, 51) est une ouverture dans le composant latéralement rigide (3, 23, 33, 43, 53).

14. Implant médical expansible (1, 21, 31, 41, 51) selon la revendication 13, comprenant en outre une ouverture de transfert (9) allant du composant latéralement rigide (3, 23, 33, 43, 53) jusqu'à la membrane (5, 25, 35, 45, 55).

15. Implant médical expansible (1, 21, 31, 41, 51) selon la revendication 1, comprenant en outre un matériau de remplissage occupant au moins la membrane (5, 25, 35, 45, 55) et fournissant le support entre la surface supérieure (6) et la surface inférieure (4).
